# EUROPEAN PATENT SPECIFICATION

(11) **EP 0 444 844 B1**
(45) Date of publication and mention of the grant of the patent: **18.01.1995**
(21) Application number: 91301480.9
(22) Date of filing: 25.02.1991
(51) Int. Cl.: C07C 405/00, A61K 31/557

(54) **New 15-dehydroxy-16-oxoprostaglandins**
15-Dehydroxy-16-oxoprostaglandine
15-Déhydroxy-16-oxoprostaglandines

(30) Priority: 26.02.1990 JP 46932/90; 11.09.1990 JP 241938/90
(43) Date of publication of application: 04.09.1991
(73) Proprietor: R-TECH UENO, LTD., Osaka-shi, Osaka-fu (JP)
(72) Inventor: Ueno, Ryuji, Nishinomiya-sho, Hyogo-ken (JP); Oda, Tomio, Sanda-shi, Hyogo-ken (JP)
(74) Representative: Atkinson, Peter Birch

(56) References cited:
- US-A- 4 170 709
- TETRAHEDRON LETTERS, no. 30, July 1977, pages 2563-2566, Pergamon Press, GB; W. BARTMANN et al.: "Darstellung von 15-Hydroxy-9-Oxo-Prosta-2-(E)-Diensäure und von 16-Hydroxy-9-Oxo-20-Homo-Prosta-2-(E)-14-(E)-Diensäure"
- STN FILE SERVER, (KARLSRUHE), FILE CA; & CHEMICAL ABSTRACTS, vol. 86, no. 7, abstract no. 43270f, Columbus, Ohio, US; & DE-A-2 608 584 & FR-A-2 302 730

## Description

The present invention relates to new prostaglandin compounds and more particularly new 15-dehydroxy-16-oxoprostaglandins.

Prostaglandins (hereinafter, prostaglandin is referred to as PG) are members of a class of organic carboxylic acid that are contained in human and most other mammalian tissues or organs and that exhibit a wide range of physiological activities. Naturally occurring PGs possess as a common structural feature the prostanoic acid skeleton: 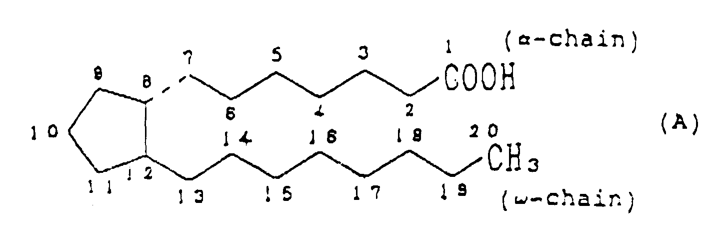
Some of synthetic analogues have somewhat modified skeletons. On the basis of the structural features of five-membered ring moiety, the PGs are classified into PGAs, PGBs, PGCs, PGDs, PGEs, PGFs, PGGs, PGHs, PGIs and PGJs. These are further classified based on whether or not the presence of unsaturated groups and oxidized groups in the chain moiety as:
- Subscript 1: - - - 13,14-unsaturated-15-OH
- Subscript 2: - - - 5,6- and 13,14-diunsaturated-15-OH
- Subscript 3: - - - 5,6- 13,14- and 17,18-triunsaturated-15-OH
In the above formula (A), for example, PGEs are compounds which have an oxo group at position 9 and a hydroxy group at position 11. PGFs are compounds which have hydroxy groups at positions 9 and 11. PGDs are compounds which have a hydroxy group at position 9 and an oxo group at position 11. PGAs are compound which have an oxo group at position 9, a hydrogen atom at position 11 and a double bond between positions 10 and 11. It has been known that PGs have various physiological activities such as anti-ulcerous, uterine-contractile, intestine-contractile, vasodilating, and diarrheic activities.

It is also known that the natural PGs are chemically unstable and very rapidly metabolized in the living body.

Compounds having an oxo group in place of a hydroxy group at position 15 of prostanoic acid skeleton and their derivatives have also been described (for example, JP-A-52753/1989, JP-A-104040/1989 and JP-A-151552/1989).

While the fact that a compound has various activities appears to be advantageous at first sight, the presence of activities which are not useful in individual cases is not desirous because they are disliked as side-effects. Therefore, it is desirous to develop compounds having only one particular activity or a limited number of activities out of various activities of PGs. Furthermore, there is a continuous demand for the compounds of this kind which have improved chemical stability and reduced rate of metabolic degradation in the living body in comparison with the natural PGs. The compounds according to the invention have been successfully synthesized as a result of extensive study seeking for such compounds.

### SUMMARY OF THE INVENTION

The present invention provides a compound of the formula: 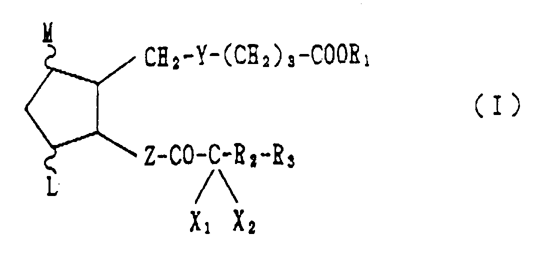
wherein
- L and M are: hydrogen atom, hydroxy, lower alkyl, hydroxy(lower)alkyl or oxo, provided that at least one of L and M is not hydrogen atom and that the five-membered ring may have one or two double bonds,
- X₁ and X₂ are: hydrogen atom, halogen atom or lower alkyl,
- Y is: -CH₂-CH₂-, -CH=CH-, -C≡C- or -CO-CH₂-,
- Z is: -CH₂-CH₂-CH₂-, -CH=CH-CH₂ or -CH₂-CH=CH-,
- R₁ is: hydrogen atom, lower alkyl, lower cycloalkyl, monocyclic aryl, monocyclic aryl(lower)alkyl or monocyclic aroyl(lower)alkyl,
- R₂ is: single bond or lower alkylene,
- R₃ is: lower alkyl which is unsubstituted or substituted with halogen, lower cycloalkyl which is unsubstituted or substituted with lower alkyl, monocyclic aryl which is unsubstituted or substituted with halogen or halo(lower)alkyl, or monocyclic aryloxy which is unsubstituted or substituted with halogen or halo(lower)alkyl,
or a pharmaceutically acceptable salt when R₁ is hydrogen atom.

### DETAILED DESCRIPTION OF THE INVENTION

In the above formula, the term "halogen" or "halo" denotes fluoro, chloro, bromo and iodo.

It is preferred that the group -CH=CH- in Y has cis configuration and the groups -CH=CH-CH₂- and -CH₂-CH=CH- in Z have trans configuration.

The term "lower" is intended to include a group having 1 to 6 carbon atoms unless otherwise specified.

The term "lower alkyl" as a group or a moiety of hydroxy(lower)alkyl, monocyclic aryl(lower)alkyl, monocyclic aroyl(lower)alkyl or halo(lower)alkyl includes saturated and straight or branched chain hydrocarbon radicals containing 1 to 6, preferably 1 to 5 and more preferable 1 to 4 carbon atoms, such as methyl, ethyl, propyl, isopropyl, butyl, isobutyl, t-butyl, pentyl and hexyl.

The term "lower alkylene" refers to the group obtainable by removing a hydrogen atom from the lower alkyl group as defined above and includes e.g. methylene, ethylene, propylene, tetramethylene, 2-methyltetramethylene, pentamethylene, hexamethylene, etc.

The term "lowercycloalkyl" refers to a cyclic group formed by cyclization of a lower alkyl group having 3 or more carbon atoms and includes, for example, cyclopropyl, cyclobutyl, cyclopentyl and cyclohexyl.

The term "halo(lower) alkyl" refers to lower alkyl group as defined above which is substituted with at least one and preferably 1 to 3 halogen atoms as defined above and includes for example, chloromethyl, bromomethyl, fluoromethyl, trifluoromethyl, 1,2-dichloromethyl, 1,2,2-trichloroethyl, chloropropyl, chlorobutyl, chloropentyl, chlorohexyl etc.

The term "hydroxy(lower)alkyl" refers to lower alkyl as defined above which is substituted with at least one hydroxy group, such as hydroxymethyl, 1-hydroxyethyl, 2-hydroxyethyl and 1-methyl-1-hydroxyethyl.

The term "monocyclic aryl" includes phenyl unsubstituted or substituted with lower alkyl substituent, for example phenyl, tolyl, xylyl, cumenyl etc.

The term "monocyclic aryloxy" refers to a group consisting of monocyclic aryl as defined above and bivalent oxygen -O- combined together, and includes, for example, phenoxy tolyloxy, xylyloxy, cumenyloxy etc.

The term " monocyclic aryl(lower)alkyl" refers to a group consisting of monocyclic aryl and lower alkyl, both as defined above, combined together, and includes, for example, benzyl, phenethyl, tolylmethyl etc.

The term "monocyclic aroyl(lower)alkyl" refers to a group consisting of monocyclic aroyl such as benzoyl unsubstituted or substituted with lower alkyl substituent and lower alkyl as defined above combined together, and includes phenacyl(benzoylmethyl), toluoylmethyl, xyloylmethyl, etc.

Suitable "pharmaceutically acceptable salt" includes conventional non-toxic salt, and may be a salt with an inorganic base, for example a metal salt such as an alkali metal salt (e.g. sodium salt, potassium salt, etc.) and an alkaline earth metal salt (e.g. calcium salt, magnesium salt, etc.), ammonium salt, a salt with an organic base, for example, an amine salt (e.g. methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino)ethane salt, monomethyl-monoethanolamine salt, procaine salt, caffeine salt, etc.), a basic amino acid salt (e.g. arginine salt, lysine salt, etc.), tetraalkylammonium salt and the like. These salts can be prepared by the conventional process, for example by use of the corresponding acid and base or by salt exchange.

The configuration of the ring and α- and/or ω-chain in the above formula (I) may be the same as or different from that in the natural prostaglandins. However, the present invention also include a mixture of a compound having natural configuration and that of unnatural configuration.

In the compounds of the present invention, when the bonds between 13-, 14- and 15-positions are saturated, a keto-hemiketal equilibrium may sometimes be formed by the formation of a hemiketal between the hydroxy group at 11-position and the keto group at 16-position.

When these tautomeric isomers are present, the ratio of the existing isomers will vary depending on the structure of other part of the molecule or the kind of possible substituents and in some cases one of the isomers is predominantly present. The present invention, however, includes both isomers, and while any compound of the invention may be represented by a structure or nomenclature of keto-type, this should be understood as a matter of mere convenience and should not be considered to be intended to exclude the compound in hemiketal type isomer.

In the present invention, individual tautomeric isomers, a mixture thereof, or optical isomers, a mixture thereof, racemic mixture and other isomers such as stereoisomers can be used for the same purpose.

### Nomenclature

Nomenclature of 15-dehydroxy-16-oxo-PG compounds herein uses the numbering system of prostanoic acid represented by the formula (A) shown above.

While the formula (A) shows the basic skeleton having twenty carbon atoms, the compounds used in the present invention are not limited to those having the same number of carbon atoms. The carbon atoms in the Formula (A) are numbered 2 to 7 on the α-chain starting towards the five membered ring from the α-carbon atom adjacent to the carboxylic carbon atom which is numbered 1, 8 to 12 on the fine membered ring in the formula (A) starting from the carbon atom on which the α-chain is attached, and 13 to 20 on the ω-chain starting counterclockwise from the carbon atom adjacent of the ring. When the number of the carbon atoms is decreased in the α-chain, the number is deleted in order starting from position 2 and when the number of the carbon atoms is increased in the α-chain, compounds are named as substituted derivatives having respective substituents in place of carboxy group (C-1) at position 2. Similarly, when the number of the carbon atoms is decreased in the ω-chain, the number is deleted in order starting from position 20 and when the number of the carbon atoms is increased in the ω-chain, compounds are named as substituted derivatives having respective substituents at position 20. Stereochemistry of the compounds is the same as that of the above formulas (A) and (B) unless otherwise specified.

Thus, 16-oxo-PGs having 10 carbon atoms in the ω-chain is named as 16-oxo-20-ethyl-PGs.

The above formula expresses a specific configuration which is most typical one, and in this specification compounds having such a configuration are expressed without any specific indication about it.

Although PGDs, PGEs and PGFs generally refer to compounds having a hydroxy group at position 9 and/or 11 of the prostanoic acid nucleus, definition of the 16-oxo-prostaglandin compounds in the present invention is extended to include compounds having another group at position 9 and/or 11. Such compounds are named as 9-dehydroxy-9-substituted or 11-dehydroxy-11-substituted compounds.

As stated above, nomenclature of 15-keto-PG compounds is based upon the prostanoic acid. These compounds, however, can also be named according to the IUPAC naming system. Some examples of the both nomenclature are shown in Examples.

The compounds of the invention can be prepared by processes shown in the following Schemes, wherein P₁, P₂, P₃, P₄, P₅, P₆ and P₇ are each protective group, A is a leaving group, Y' is -CH=CH-, L' is lower alkyl, R₁' is lower alkyl or monocyclic aryl(lower)alkyl, and X₁, X₂, R₂ and R₃ are the same as defined above. 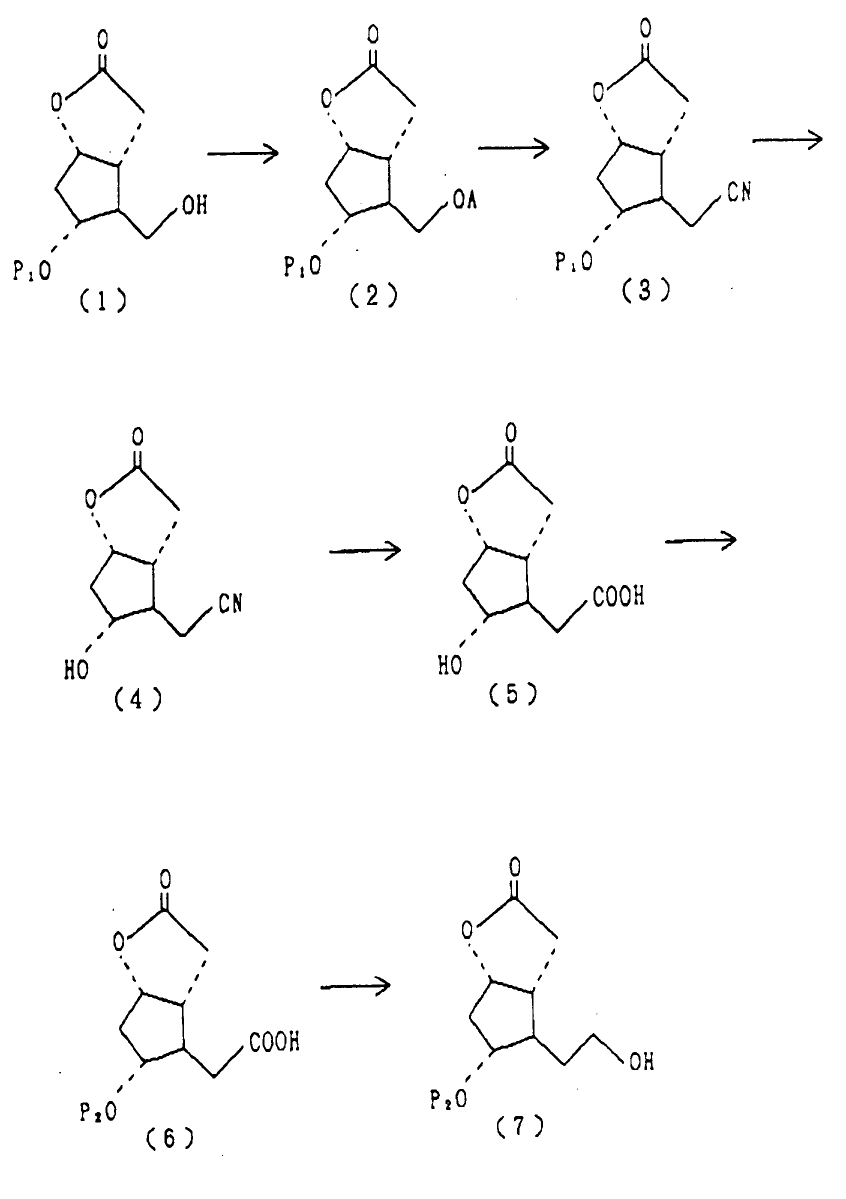 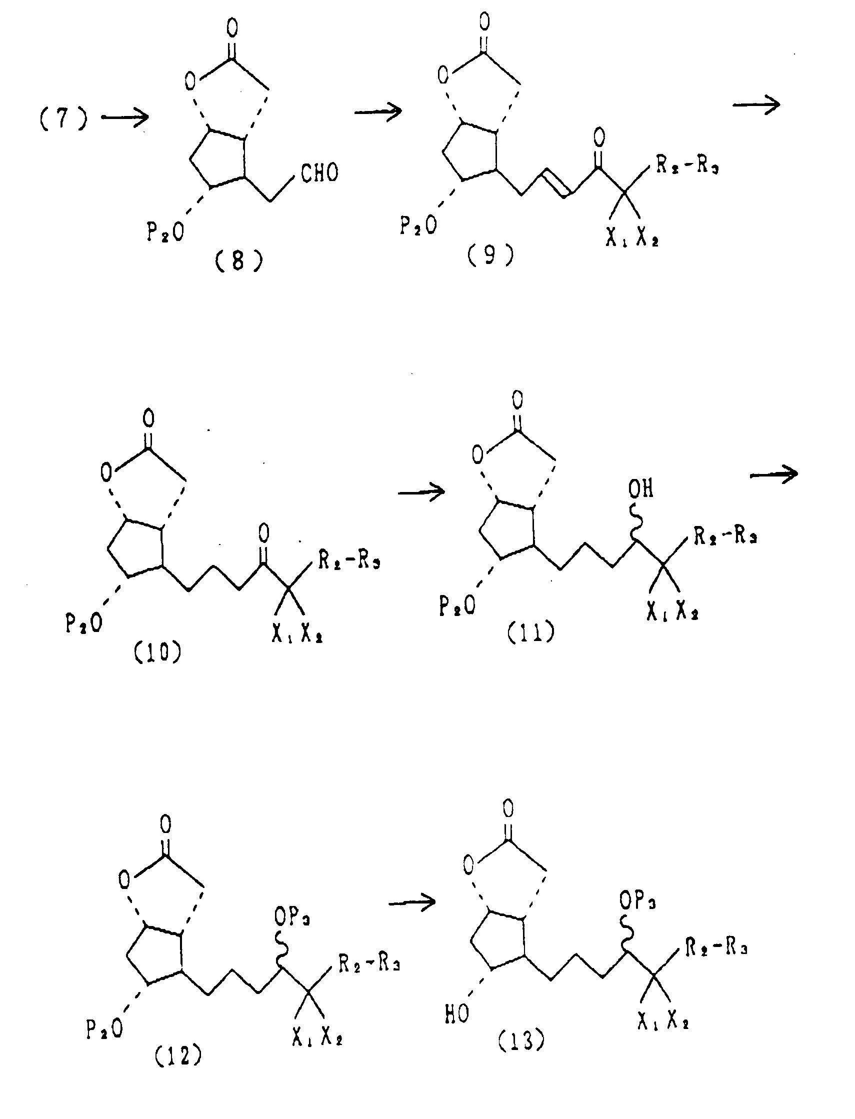 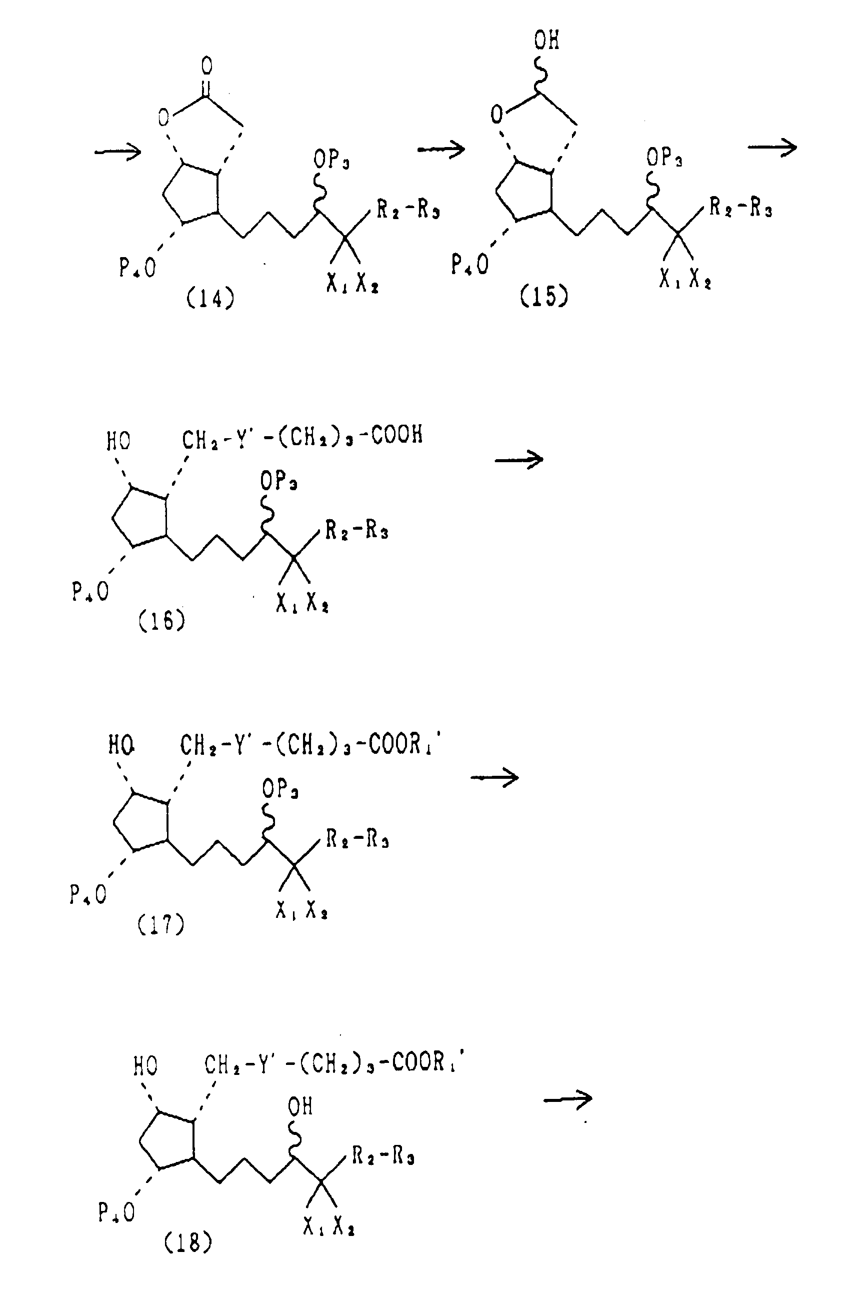 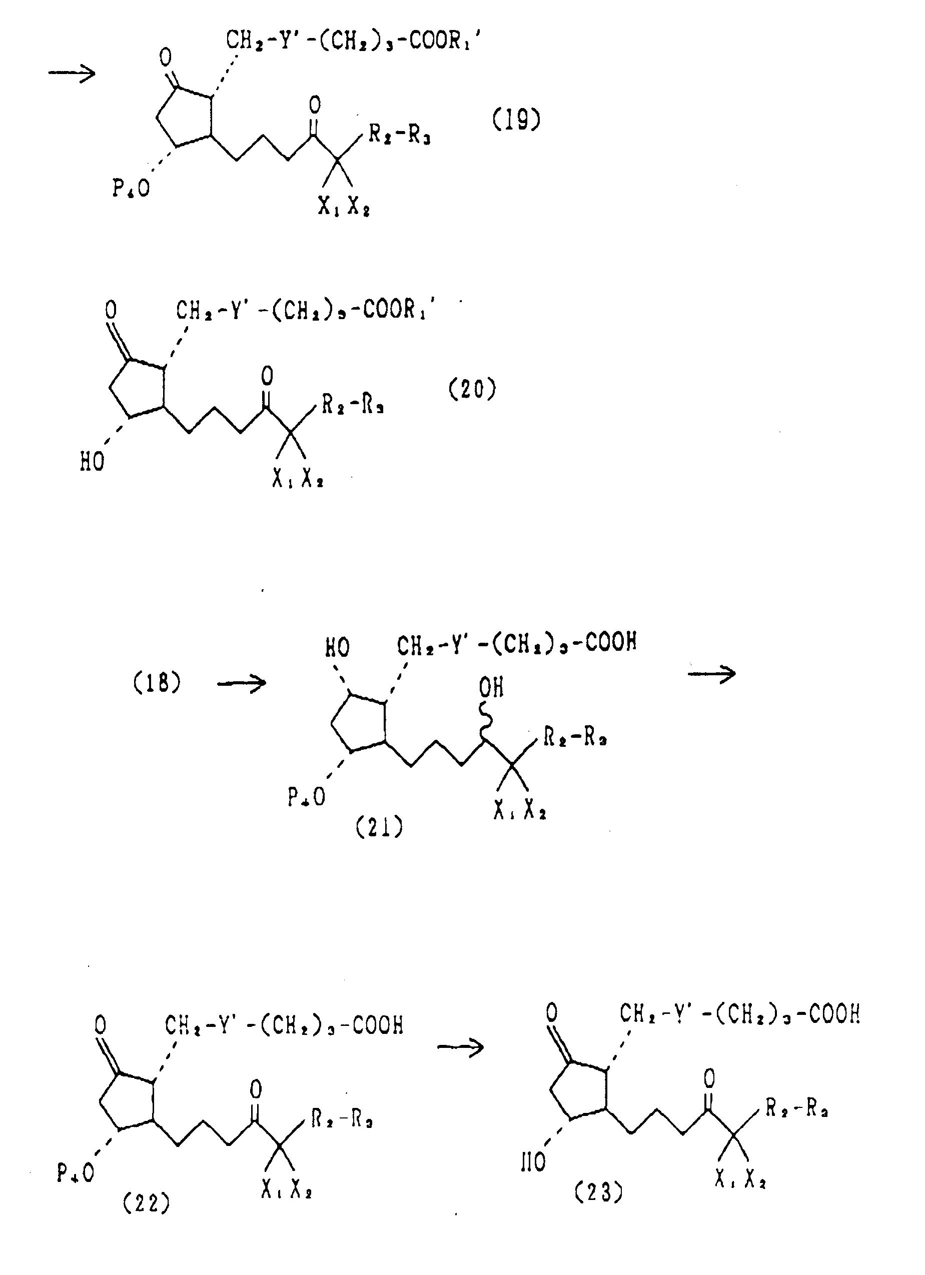 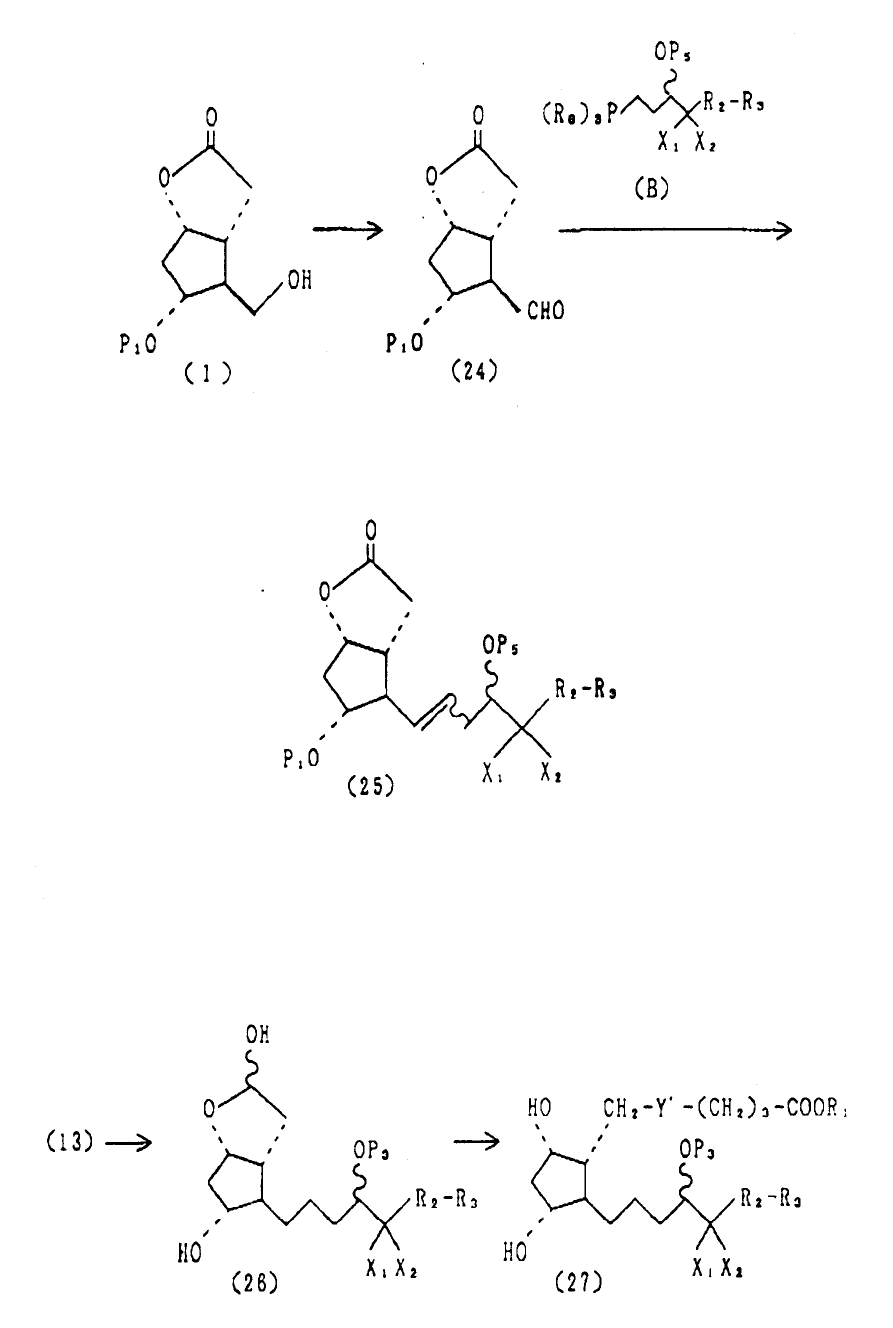 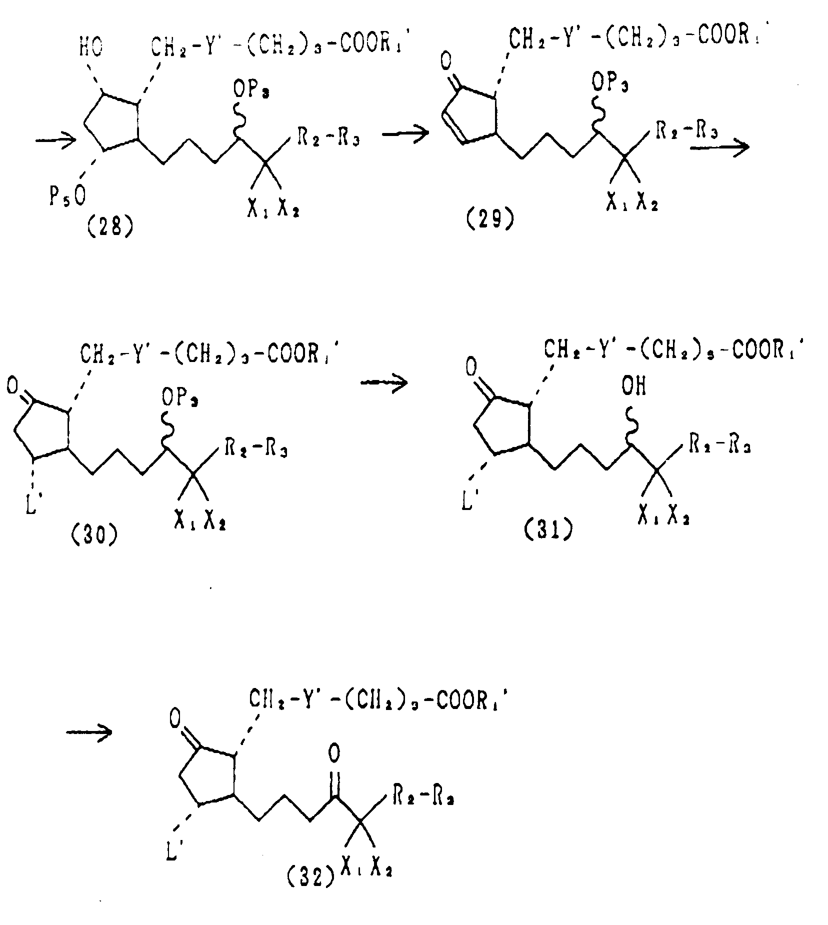 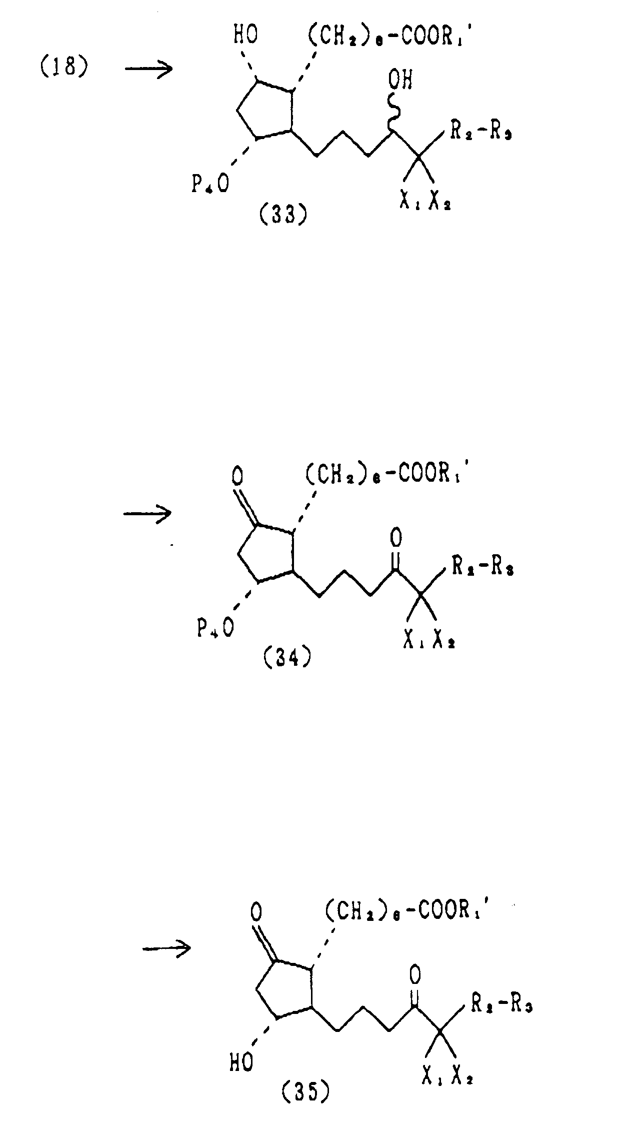 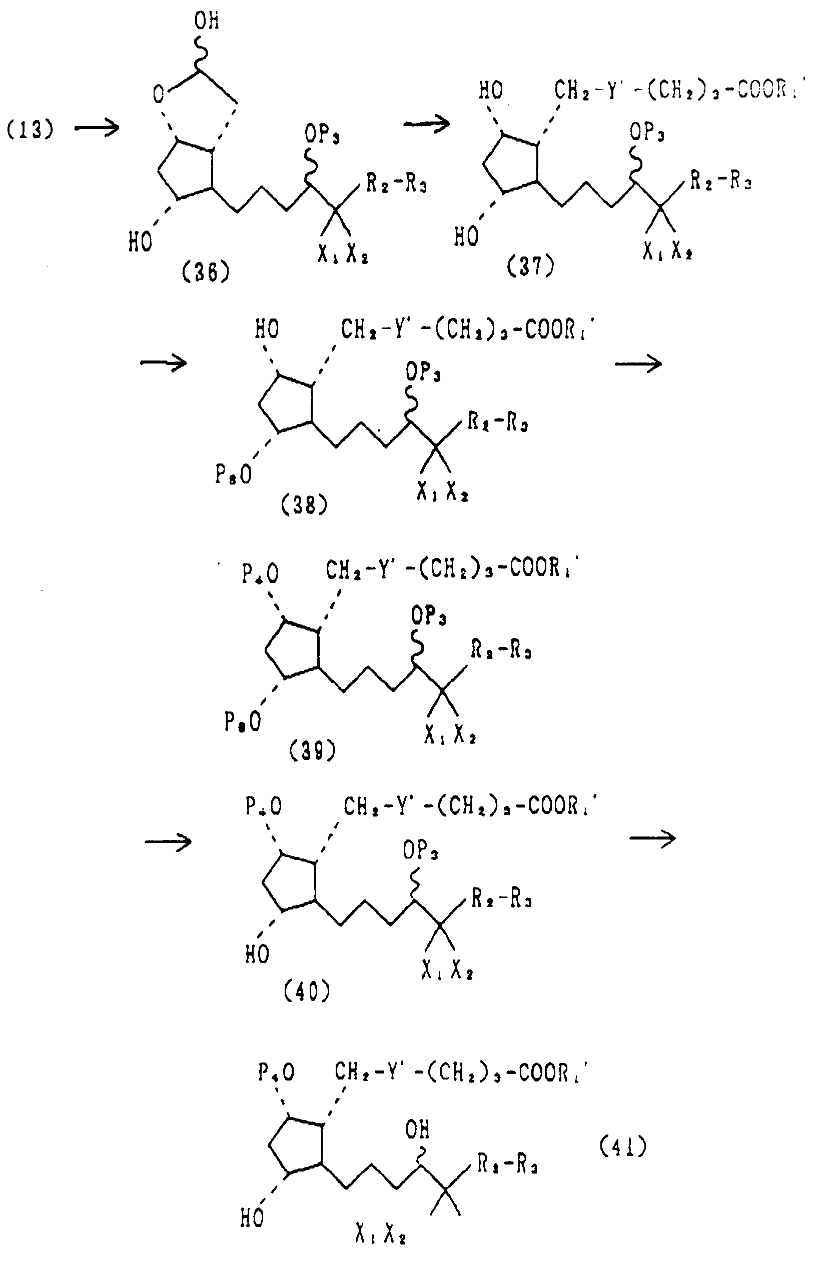 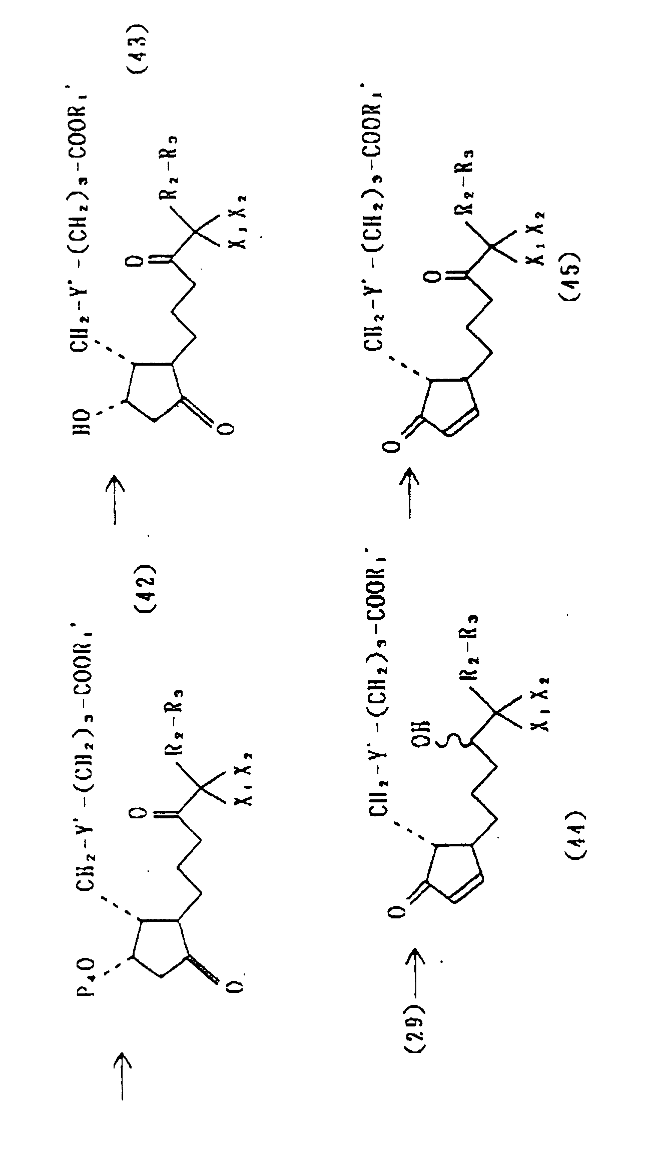 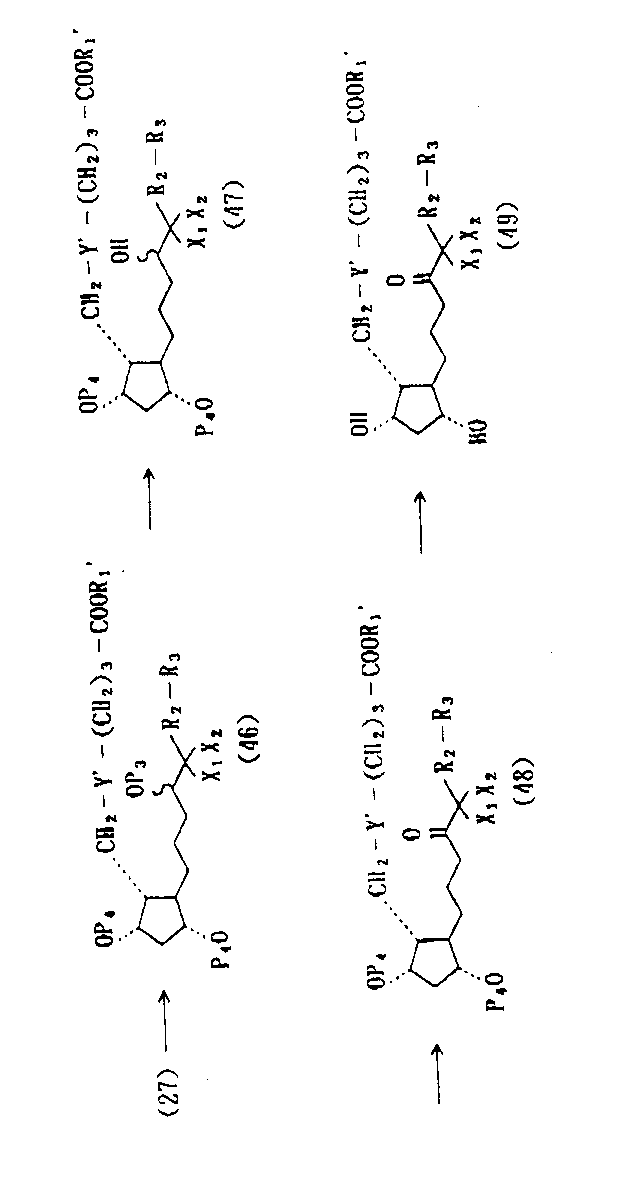 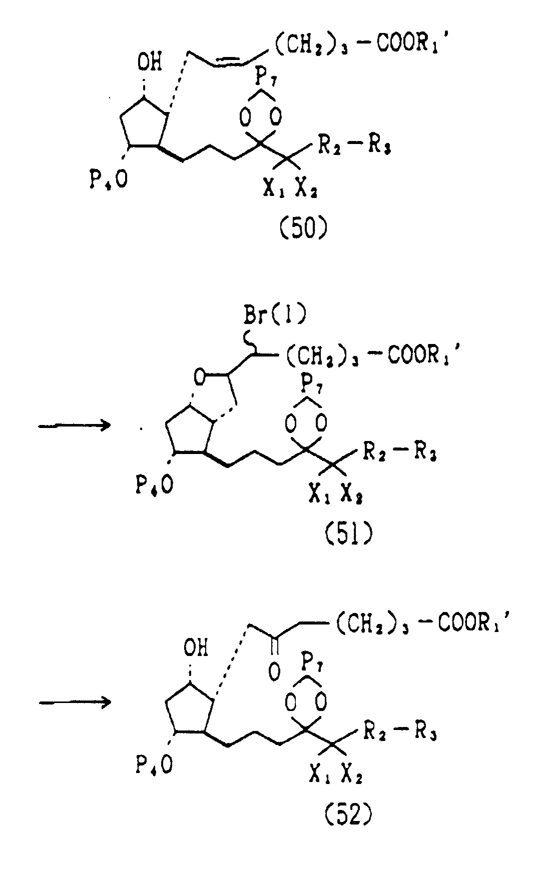 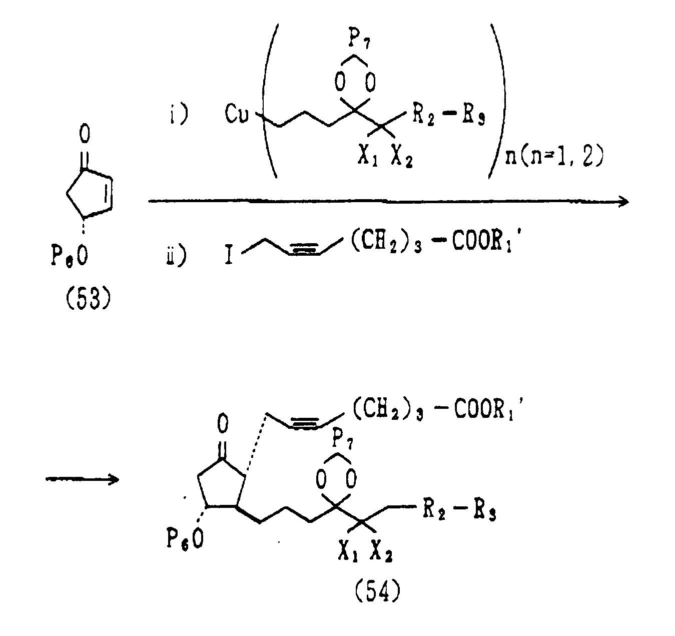
Referring to the above Schemes, the process steps from the compound (1) to the compound (7) show a reaction for elongation of the carbon chain. In the first place, a leaving group (such as tosyl) is introduced to Corey lactone (1) having an appropriate protecting group (for example, 4-phenylbenzoyl) (commercially available) to form the compound (2), which is reacted with a compound generating cyamide ion to give the nitrile (3). Deprotection of it produces the compound (4), the cyano group in which is hydrolyzed to form the compound (5). After introducing a protective group (preferably acyl such as acetyl) to give the compound (6), the carboxy group is reduced to yield the compound (7) that is a compound in which the number of the carbon atoms in the chain is increased by 1.

The compound (7) is oxidized (for example, by Collins oxidation) into the compound (8), which is reacted with (2-oxoalkyl)phosphonate having desired X₁, X₂, R₂ and R₃ to yield the compound (9). As the phosphonate, (3,3-difluoro-2-oxoalkyl)phosphonate (when X₁ and X₂ are fluorine), (3,3-dimethyl-2-oxoalkyl)phosphonate (when X₁ and X₂ are methyl) or (2-oxo-4-phenylbutyl)phosphonate (when R₂ is methylene and R₃ is phenyl) may be used. If a 14,15-dihydro compound is desired, the compound (9) is subjected to reduction of the double bond to form the compound (10), and of which oxo group is reduced to give the compound (11), of which hydroxy group is protected to give the compound (12). The acyl protecting group for the hydroxy group at position 11 is removed to give the compound (13) and another protecting group (such as tetrahydropyranyl) is introduced to form the compound (14), of which the lactone ring is then reduced to the corresponding lactol (15). To this is introduced an alpha-chain by Witig reaction to produce the compound (16), which is esterified to the compound (17) and protection group of the hydroxy group at position 16 is removed to give the compound (18). Oxidation of the hydroxy groups at position 16 and 9 giving the compound (19) and deprotection of the hydroxy group at position 11 gives the desired compound (20). In the above preparation, when the reduction of the compound (9) to the compound (10) is omitted, the compound wherein Z is -CH₂-CH=CH= is obtained. The compound wherein Z is -CH=CH-CH₂ can be obtained from Corey lactone (1) which is oxidized, without the reaction for elongation of the carbon chain, to give the aldehyde (24), which is reacted with a (3-hydroxyalkyl)triarylphosphonium halide (B) to give the compound (25). This compound is processed in a manner similar to that for the preparation of the compound (12) to produce the desired compound. This is a mixture of cis- and trans-compounds in respect of the double bond at positions 13 and 14, and can be separated by suitable conventional means. The compound wherein Y is -CH₂-CH₂- can be obtained by using appropriately selected alpha-chain introducing agent or by reducing the compound (18), followed by oxidation and deprotection, via the dihydro compound (33) and the diketone (34). The compound wherein R₁ is a hydrogen atom is obtained after hydrolysis of the compound (20).

In another process, the compound (18) is hydrolyzed to the compound (21), which is oxidized with an oxidizing agent, for example chromic acid, to the compound (22) and then the protecting group of the hydroxy group at position 11 is removed to produce the desired compound (23).

In a further process, in which the compound (I) wherein L is other than a hydroxy group (for example, L is lower alkyl) is desired, the lactone ring of the compound (13) is reduced to form the compound (26), to which the alpha-chain is introduced by Witig reaction to give the compound (27). The hydroxy group at position 11 is protected with, for example, a monocyclic arylsulfonyl group to give the compound (28), which is oxidized (by, for example, Jones oxidation) to be the compound (29). This is reacted with a lower alkyl copper complex to 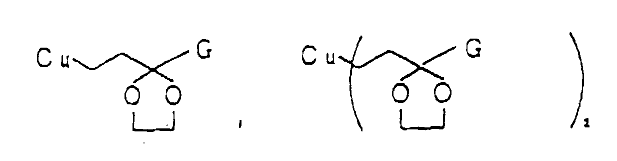
wherein G is alkyl
yield the compound (30), of which protection group for the hydroxy group at position 16 is removed. The obtained alcohol (31) is oxidized to produce the desired compound (32).

The PGD-type compounds can be obtained by reducing the compound (13) to the lactol (36), to which the alpha-chain is introduced to form the diol (37). This is converted to the 11-protected compound (38), 9,11-deprotection compound (39), 9-protected compound (40), 16-deprotected compound (41) and then to diketone (42), which at position 9 is removed to produce the compound (43).

The PGA-type compounds can be obtained by oxidation of the 16-deprotected compound (44), which is obtained from the compound (29), to the compound (45).

The PGF-type compounds can be obtained after introduction of a protective group to the compound (27) to give the compound (46), which is deprotected at the side chain to form the compound (47), oxidized to the compound (48) and then deprotected to produce the compound (49). The 6-keto compounds are produced by the reaction with the 5,6-ethylenic compound (50) with N-bromosuccinimide or iodine to form the compound (51), which is treated with DBU (1,8-diazabicyclo-[5.4.0]undec-7-ene). The 5,6-dehydro compounds (i.e. acetylenic compounds) are obtained by the reaction with the copper enolate, generated from the compound (53) and copper complex, with 6-alkoxycarbonyl-1-iodo-2-hexyne.

Since the compounds (I) have not only improved chemical stability and reduced rate of metabolic degradation but also desired activity or activities out of a wide range of activities of PGs (cf. for example Kirk-Othmer ENCYCLOPEDIA OF CHEMICAL TECHNOLOGY, 3rd. Ed., Supplement Vol., P.721) with less or almost no activity or activities undesirous for human or other animal in the situation in which the compounds are administered, the said compounds are useful as new PG derivatives having selected activity or activities. Such activity or activities can be measured by the conventional pharmacological assay methods which have been used for evaluating the activities of natural and synthetic PGs. In addition, the compounds of the invention are useful as stable reference agents having activities of PGs and usable in comparative biochemical test.

### Example

The practical embodiments for the production of the invention are illustratively shown in the following Examples.

### Example 1

### Preparation of 15-dehydroxy-17,17-difluoro-13,14-dihydro-16-oxo-PGE₂ (20) methyl ester [The IUPAC nomenclature:methyl (Z)-7-[(1R)-(2R,3R)-2-(5,5-difluoro-4-oxooctyl)-3-hydroxy-5-oxocyclopentyl]hept-5-enoate]

### 1-1) Preparation of (1S,5R,6R,7R)-6-cyanomethyl-7-hydroxy-2-oxabicyclo[3.3.0]octan-3-one (4)

P-toluenesulfonyl chloride (30.3 g) was added to a solution of commercially available (-)-Corey lactone (1) (15.0g) in pyridine, and the resultant mixture was stirred for 15 hours.

The reaction mixture was worked up with the conventional procedure to give the crude tosylate (2).

The tosylate (2) was dissolved in dimethyl sulfoxide and sodium cyanide (3.92g) was added thereto, and the resultant mixture was stirred at 60 to 70°C for 2 hours. The reaction mixture was worked up with the conventional procedure to give the crude cyano compound (3). The crude cyano compound (3) was dissolved in methanol, and potassium carbonate (2.76g) was added thereto, and the resultant mixture was stirred for 15 hours. The reaction mixture was concentrated under reduced pressure, and the obtained residue was chromatographed on a silicagel column to give the titled compound (4).
Yield: 3.93g (51%)

### 1-2) Preparation of 2-{(6R)-(1S,5R,7R)-7-acetoxy-3-oxo-2-oxabicyclo[3.3.0]octyl}-acetic acid (6)

(1S,5R,6R,7R)-6-Cyanomethyl-7-hydroxy-2-oxabicyclo[3.3.0]octan-3-one (4) (1.25g) was dissolved in 1N sodium hydroxyde solution and the resultant mixture was stirred at 100 to 110 °C. The reaction mixture was allowed to be cool, neutralized with hydrochloric acid and concentrated under reduced pressure. To the obtained residue were added ethyl acetate and methanol, and insoluble materials were removed by filtration. The filtrate was concentrated under reduced pressure to give the crude carboxylic acid (5). To the carboxylic acid (5) were added acetic anhydride (20ml) and pyridine (10ml), and the resultant mixture was stirred for 15 hours. The reaction mixture was concentrated under reduced pressure and the obtained residue was treated with 1N hydrochloric acid and the resultant mixture was stirred for 1 hour. The reaction mixture was worked up with the conventional procedure to give the crude titled compound (6).

### 1-3) Preparation of (1S,5R,6R,7R)-7-acetoxy-6-(2-hydroxy-ethyl)-2-oxabicyclo[3.3.0]octan-3-one (7)

The product obtained in 1-2), namely 2-[(6R)-(1S,5R,7R)-7-acetoxy-3-oxo-2-oxabicyclo[3.3.0]octyl]acetic acid (6), was dissolved in ethyl actate and the resultant solution was cooled to 0°C. Boron dimethyl sulfide complex (0.65ml) was added and the solution was stirred for 3 hours at room temperature. Methanol (6ml) was added to the reaction mixture and the resultant mixture was concentrated under reduced pressure. The obtained residue was subjected to silicagel column chromatography to give the titled compound (7).
Yield: 0.803g (51%, calculated from Compound (4))

### 1-4) Preparation of (1S,5R,6R,7R)-7-acetoxy-6-[(E)-5,5-difluoro-4-oxo-2-octenyl]-2-oxabicyclo[3.3.0]octan-3-one (9)

A solution of oxalyl chloride (0.90ml) in methylene chloride was cooled to -78°C and dimethyl sulfoxide (DMSO) (1.64ml) was added thereto.

To the resultant mixture was added (1S,5R,6R,7R)-7-acetoxy-6-(2-hydroxyethyl)-2-oxabicyclo[3.3.0]octan-3-one (7) (1.77g) in methylene chloride. After 30 minutes, the resultant solution was warmed to -30°C. Trimethylamine (3.28ml) was added and the mixture was stirred for additional 30 minutes. To the reaction mixture was added saturated ammonium chloride solution. The resultant mixture was worked up with the conventional procedure to give the crude aldehyde product (8).

To a solution of thallium(I) ethoxide (1.29g) in tetrahydrofuran (THF) was added a solution of dimethyl (3,3-difluoro-2-oxohexyl)phosphonate (1.39g) in THF. The resultant solution was cooled to 0°C, followed by addition of a solution of the aldehyde (8) in THF. The resultant mixture was stirred for 15 hours, and neutralized with acetic acid. An aqueous potassium iodide solution was added and insoluble matters were removed by filtration. The filtrate was worked up with the conventional procedure and the obtained residue was subjected to silicagel column chromatography to give the title compound (9).
yield: 0.967g (54%)

### 1-5) Preparation of (1S,5R,6R,7R)-7-acetoxy-6-{5,5-difluoro-4-(RS)-hydroxyoctyl}-2-oxabicyclo[3.3.0]octan-3-one (11)

Palladium on charcoal (0.200g) was added to a solution of (1S,5R,6R,7R)-7-acetoxy-6-[(E)-5,5-difluoro-4-oxo-2-octenyl]-2-oxabicyclo[3.3.0]octan-3-one (9) (1.55g) in ethyl acetate. The resultant mixture was stirred for 15 hours under a hydrogen atmosphere. The reaction mixture was filtered and the filtrate was concentrated under reduced pressure to give the crude ketone (10).

Sodium borohydride (0.169g) was added to a solution of crude ketone (10) in methanol. After 30 minutes, acetic acid was added and the resultant mixture was worked up with the conventional procedure. The obtained crude product was subjected to silicagel column chromatography to give the titled compound (11).
Yield: 1.52g (97%)

### 1-6) Preparation of (1S,5R,6R,7R)-6-{4(RS)-t-butyl dimethylsiloxy-5,5-difluorooctyl}-7-hydroxy-2-oxabicyclo[3.3.0]octan-3-one (13)

Imidazol (1.78g) and t-butyldimethylsilyl chloride (1.97g) were added to a solution of (1S,5R,6R,7R)-7-acetoxy-6-{5,5-difluoro-4-(RS)-hydroxyoctyl}-2-oxabicyclo[3.3.0]octan-3-one (11) (1.52g) in N,N-dimethyl formamide. The resultant solution was stirred for 3 days.

The reaction mixture was worked up with the conventional procedure to give the crude silyl product (12). The obtained silyl product (12) was dissolved into methanol, followed by addition of potassium carbonate (0.60g). The resultant mixture was stirred for 2 hours. The reaction mixture was worked up with the conventional procedure and the obtained product was subjected to silicagel column chromatography to give the titled compound (13).
Yield: 1.63g (89%)

### 1-7) Preparation of (1S,5R,6R,7R)-6-{4(RS)-t-butyldimethylsiloxy-5,5-difluorooctyl}-7-tetrahydropyranyloxy-2-oxabicyclo[3.3.0]octan-3-one (14)

To a solution of (1S,5R,6R,7R)-6-[4(RS)-t-butyldimethyl-siloxy-5,5-difluorooctyl]-7-hydroxy-2-oxabicyclo[3.3.0]octan-3-one (13) (1.63g) in methylene chloride were added dihydropyran (1.70ml) and p-toluene sulfonic acid monohydrate (20mg). After 30 minutes, the resultant mixture was worked up with the conventional procedure and the obtained residue was subjected to silicagel column chromatography to give the titled compound (14).
Yield: 1.93g (99%)

### 1-8) Preparation of methyl (Z)-7-[(1R)-(2R,3R,5S)-2-{4(RS)-t-butyldimethylsiloxy-5,5-difluorooctyl}-5-hydroxy-3-tetrahydropyranyloxycyclopentyl]hept-5-enoate (17)

Diisobutylaluminium hydride (DIBAL-H) (1.0M, 11.5ml) was added to a solution of (1S,5R,6R,7R)-6-{4(RS)-t-butyl-dimethylsiloxy-5,5-difluorooctyl}-7-tetrahydropyranyloxy-2-oxabicyclo[3.3.0]octan-3-one (14) (1.93g) in toluene. After 30 minutes, methanol and a saturated Rochelle salt solution were added and the resultant mixture was worked up with the conventional procedure to give the crude lactol (15).

To a suspension of (4-carboxybutyl)-triphenylphosphonium bromide (6.80g) in THF was added dropwise a solution of potassium t-butoxide (1.0M, 30.7ml). The resultant mixture was stirred for 15 minutes. The reaction mixture was cooled to -40°C and a solution of the lactol (15) prepared above in tetrahydrofuran was added thereto. The reaction temperature was kept at 25°C while stirring for 15 hours and worked up with the conventional procedure to give the crude carboxylic acid (16).

To a solution of the crude carboxylic acid (16) in ether was added a solution of diazomethane in ether prepared with the ordinal method. The reaction mixture was concentrated under reduced pressure, and the obtained residue was subjected to column chromatography with silica gel to give the titled compound (17).
Yield: 1.90g (82%)

### 1-9) Preparation of methyl (Z)-7-[(1R)-(2R,3R,5S)-2-{5,5-difluoro-4(R,S)-hydroxyoctyl}-5-hydroxy-3-tetrahydropyranyloxycyclopentyl]hept-5-enoate (18)

To a solution of methyl (Z)-7-[(1R)-(2R,3R,5S)-2-{4(RS)-t-butyldimethylsiloxy-5,5-difluorooctyl}-5-hydroxy-3-tetrahydropyranyloxycyclopentyl]hept-5-enoate (17) (1.90g) in tetrahydrofuran was added tetrabutylammonium fluoride in tetrahydrofuran (1.0M, 15.7ml). The resultant mixture was stirred at room temperature for 3 days. The reaction mixture was concentrated under reduced pressure and the obtained residue was subjected to silicagel column chromatography to give the titled compound (18).
Yield: 1.16g (75%)

### 1-10) Preparation of methyl (Z)-7-[(1R)-(2R,3R)-2-(5,5-difluoro-4-oxooctyl)-5-oxo-3-tetrahydropyranyloxycyclopentyl]hept-5-enoate (19)

A solution of oxalyl chloride (0.165ml) in methylene chloride was cooled to -78°C and dimethyl sulfoxide (DMSO) (0.30ml) was added thereto.

To the above solution was added a solution of methyl (Z)-7-[(1R)-(2R,3R,5S)-2-{5,5-difluoro-4(RS)-hydroxyoctyl}-5-hydroxy-3-tetra-hydropyranyloxycyclopentyl]hept-5-enoate (18) (0.244g) in methylene chloride. The resultant mixture was warmed to -25°C and stirred for 1 hour. Triethylamine (0.60ml) was added thereto and the reaction mixture was stirred for additional 30 minutes, poured into 1N hydrochloric acid, and then worked up with the conventional procedure. The obtained product was subjected to silicagel column chromatography to give the titled compound (19).
Yield: 0.20g (83%)

### 1-11) Preparation of 15-dehydroxy-17,17-difluoro-13,14-dihydro-16-oxo-PEG₂ methyl ester [methyl (Z)-7-{(1R)-(2R,3R)-2-(5,5-difluoro-4-oxooctyl)-5-oxo-3-hydroxycyclopentyl}hept-5-enoate (20)]

Methyl (Z)-7-[(1R)-(2R,3R)-2-{5,5-difluoro-4-oxooctyl}-5-oxo-3-tetrahydropyranyloxycyclopentyl]hept-5-enoate (19) (0.20g) was dissolved in a mixed solvent of acetic acid, water and tetrahydrofuran (4:2:1) and the resultant solution was stirred at 45 to 50°C for 3 hours. The reaction mixture was concentrated under reduced pressure and the obtained product was subjected to silicagel column chromatography and further to medium pressure chromatography on Rober column (Merck & Co.,Inc. ODS, type B) to give the titled compound (20).
Yield: 0.124g (75%)
Compound (20) (X₁=X₂=F, R₂-R₃=propyl, R'₁=methyl)
¹HNMR (CDCl₃) δ 0.98(t,3H,J=7Hz),1.1-2.80 (m,22H),3.11(m,1H),3.68(s,3H),4.12-4.27(m,0.73H),4.32-4.47 (m,0.27H),5.25-5.54(m,2H)
MS (DI-EI)m/z402(M⁺),384(M⁺-H₂O),368(M⁺-HF-H₂O),353(M⁺ -OCH₃-H₂O),309(M⁺-C₄H₇F₂)

### Example 2

### Preparation of 15-dehydroxy-17,17,-difluoro-13,14-dihydro-16-oxo-PGE₂ (23) [The IUPAC nomenclature:(Z)-7-{(1R)-(2R,3R)-2-(5,5-difluoro-4-oxooctyl)-5-oxo-3-hydroxycyclopentyl}hept-5-enoic acid]

### 2-1) Preparation of (Z)-7-{(1R)-(2R,3R)-2-(5,5-difluoro-4-oxooctyl)-5-oxo-3-tetrahydropyranyloxycyclopentyl}hept-5-enoic acid (22)

1N Sodium hydroxide solution (4.8ml) was added to a solution of methyl (Z)-7-[(1R)-(2R,3R,5S)-2-{5,5-difluoro-4(RS)-hydroxyoctyl}-5-hydroxy-3-tetrahydro-pyranyloxycyclopentyl]hept-5-enoate (18) (0.457g) in methanol. The resultant mixture was stirred for 4 hours and treated in the conventional manner to give dialcohol (21).

Chromic acid (3.67g) was added to pyridine (5.93ml) in methylene chloride. The resultant mixture was stirred for 1 hour and celite was added thereto. A solution of the diol (21) in methylene chloride was added and the resultant mixture was stirred for 30 minutes. Then, sodium bisulfate (30g) was added thereto. The reaction mixture was worked up with the conventional procedure to give a crude product, which was subjected to procedure silicagel (Mallincklodt, CC-4) column chromatography to give the titled compound (22).
Yield: 0.231g (53%)

### 2-2) Preparation of 15-dehydroxy-17,17-difluoro-13,14-dihydro-16-oxo-PGE₂ (23) [The IUPAC nomenclature: (Z)-7-{(1R)-(2R,3R)-2-(5,5-difluoro-4-oxooctyl)-5-oxo-3-hydroxycyclopentyl}hept-5-enoic acid]

A solution of (Z)-7-[(1R)-(2R,3R)-2-(5,5-difluoro-4-oxooctyl)-5-oxo-3-tetrahydropyranyloxycyclopentyl]hept-5-enoic acid (22) (0.231g) in a mixed solvent of acetic acid, water and tetrahydrofuran (4:2:1) was stirred at 45°C for 3.5 hours. The reaction mixture was concentrated under reduced pressure and the obtained residue was subjected to medium pressure chromatography on a Rober column (Merck, & Co., Inc., ODS, type B) to give the titled compound (23).
Yield: 0.110g (58%)
Compound (23) (X₁=X₂=F, R₂-R₃=propyl)
¹HNMR (CDCl₃) δ 1.00(t,3H,J=7Hz),1.10-2.80(m,22H),4.12 -4.27(m,0.71H),4.32-4.46(m,0.29H),5.27-5.55(m,2H),4.0-6.5 (br.s,2H).
MS (DI-EI)m/z388(M⁺),370(M⁺-H₂O).

### Example 3

Preparation of 15-dehydroxy-17,17-difluoro-13,14-dihydro-16-oxo-PGE₂ isopropyl ester (20) [The IUPAC nomenclature: Isopropyl (Z)-7-{(1R)-(2R,3R)-2-(5,5-difluoro-4-oxooctyl)-3-hydroxy-5-oxocyclopentyl}hept-5-enoate]

### 3-1) Preparation of Isopropyl (Z)-7-[(1R)-(2R,3R,5S)-2-{4(R,S)-t-butyldimethylsiloxy-5,5-difluorooctyl}-5-hydroxy-3-tetrahydropyranyloxycylopentyl]hept-5-enoate (17)

To a solution of the crude carboxylic acid (16) in acetonitrile were added isopropyl iodide (0.85ml) and 1,8-diazabicyclo[5.4.0]undec-7-ene (DBU) (1.29ml). The resultant mixture was kept at 60 to 65°C for 2 hours. The crude product obtained after the usual work-up was subjected to silicagel column chromatography, to give the titled compound (17).
Yield: 1.1g (87%)

### 3-2) Preparation of isopropyl (Z)-7-[(1R)-(2R,3R,5S)-2-{4(R,S)-hydroxy-5,5-difluorooctyl}-5-hydroxy-3-tetrahydropyranyloxycylopentyl]hept-5-enoate (18)

To a solution of the compound (17) (1.1g) in THF was added tetrabutylammonium fluoride (1M THF, 5.5ml). The resultant mixture was stirred for 1 hour and 20 minutes. The product obtained after the usual work-up was subjected to silicagel column chromatography to give the titled compound (18).
Yield: 0.906g (100%)

### 3-3) Preparation of isopropyl (Z)-7-{(1R)-(2R,3R,5S)-2-(5,5-difluoro-4-oxooctyl)-5-oxo-3-tetrahydropyranyloxycylopentyl}hept-5-enoate (19)

A solution of oxalyl chloride in methylene chloride (2M, 3.5ml) was cooled to -78°C, followed by addition of DMSO (1.1ml). A solution of the compound (18) (0.906g) in methylene chloride (11ml) was added dropwise. The resultant mixture was stirred at the range of -35 to -25°C for 1.5 hours, and triethylamine (2.1ml) was added dropwise. After 20 minutes, 1N hydrochloric acid was added. The crude product obtained after the usual work-up was subjected to silicagel column chromatography to give the titled compound (19).
Yield: 0.785g (87.7%)

### 3-4) Preparation of isopropyl (Z)-7-{(1R)-(2R,3R)-2-(5,5-difluoro-4-oxooctyl)-5-oxo-3-hydroxycylopentyl}-hept-5-enoate (20)

A solution of the compound (19) (0.785g) in a mixed solvent of acetic aid, THF and water (3:1:1, 70ml) was kept at 50°C for 4.5 hours. The crude product obtained after the usual work-up was subjected to silicagel column chromatography to give the titled compound (20).
Yield: 0.335g
Compound (20) (X₁=X₂=F, R₂-R₃=propyl, R'₁=methyl)
¹HNMR (CDCl₃) δ 0.94(t,3H,J=7.4Hz),1.20(d,6H,J=6.2Hz), 1.3-2.9(m,22H),4.17(m,1H),4.98(hept,1H,J=62Hz),5.22-5.52 (m,2H).
MS (DI-ZI) m/z 430(M⁺),412(M⁺-H₂O),371(M⁺-C₃H₇O), 353(M⁺-C₃H₇O-H₂O).

### Example 4

### Preparation of 11,15-didehydroxy-17,17-difluoro-13,14-dihydro-11-methyl-16-oxo-PGE₂ methyl ester (32) [The IUPAC nomenclature: methyl (Z)-7-{(1R,2S,3R)-2-(5,5-difluoro-4-oxooctyl)-3-methyl-5-oxocylopentyl}hept-5-enoate]

### 4-1) Preparation of {1S,3(R,S),5R,6R,7R}-6-{4(R,S)-t-butyldimethylsiloxyoctyl-5,5-difluorooctyl}-3,7-dihydroxy-2-oxabicyclo[3.3.0]octane (26)

A solution of (1S,5R,6R,7R)-6-{4(R,S)-t-butyldimethylsiloxy-5,5-difluorooctyl}-7-hydroxy-2-oxabicyclo[3.3.0]octan-3-one (13) (1.06g) in toluene was cooled to -78°C and DIBAL-H (1.5M, 7.56ml) was added dropwise thereto. After 30 minutes methanol (8ml) was added. The reaction mixture was worked up with the conventional manner to give the lactol (26).

### 4-2) Preparation of methyl (Z)-7-[(1R)-(2R,3R,5S)-2-{(4(R,S)-t-butyldimethylsiloxy-5,5-difluorooctyl}-3,5-dihydroxycylopentyl]hept-5-enoate (27)

To a suspension of (4-carboxybutyl)triphenylphosphonium bromide (6.7g) in THF (5ml) was added dropwise potassium t-butoxide (1.0M, in THF solution) (30.2ml). The resultant mixture was stirred at room temperature for 30 minutes, and then cooled to -40°C. A solution of lactol (26) in THF (15ml) was added thereto. The resultant mixture was stirred overnight at -20°C. The crude carboxylic acid obtained after the usual work-up was esterified with diazomethane. The obtained product was subjected to silicagel column chromatography to give the diol (27).
Yield: 1.12g (85%)

### 4-3) Preparation of methyl (Z)-7-[(1R)-(2R,3R,5S)-2-{(4(R,S)-t-butyldimethylsiloxy-5,5-difluorooctyl}-5-hydroxy-3-(p-toluenesulfoxy)cylopentyl]hept-5-enoate (28)

A solution of the diol (27) (0.574g) in pyridine was cooled to -20°C, followed by addition of p-toluenesulfonyl chloride (2.1g). The resultant mixture was stirred for 1 hour at -20°C and for additional 2 hours at 0°C. The crude product obtained after the usual work-up was subjected to silicagel column chromatography to give the monotosylate (28).
Yield: 0.465g (63%)

### 4-4) Preparation of methyl (Z)-7-[(1R,2R)-2-{4(R,S)-t-butyldimethylsiloxy-5,5-difluorooctyl}-5-oxocylopent-3-enyl]hept-5-enoate (31)

A solution of the monotosylate (15) (0.465g) in acetone (20ml) was cooled to -30°C and Jones reagent (0.9ml) was added dropwise thereto. The resultant mixture was stirred at the range of -20 to 10°C for 50 minutes, followed by addition of isopropanol (0.9ml). After stirring for 20 minutes, the reaction mixture was worked up with the conventional procedure. The obtained crude product was subjected to silicagel column chromatography to give the α,β-unsaturated ketone (29).
Yield: 0.201g (71%)

### 4-5) Preparation of methyl (Z)-7-[(1R,2S,3R)-2{4(R,S)-t-butyldimethylsiloxy-5,5-difluorooctyl}-3-methyl-5-oxocylopentyl]hept-5-enoate (30)

Copper (II) iodide (0.313g) was added to anhydrous ether (15ml). The resultant suspension was cooled to 0°C and methyl lithium (1.4M, 2.35ml) was added thereto. After the resultant mixture became colorless and clear, a solution of the α,β-unsaturated ketone (29) in ether (15ml) was added thereto. The crude product obtained after the usual work-up was subjected to silicagel column chromatography to give the titled compound (30).
Yield: 0.201g (71%)

### 4-6) Preparation of methyl (Z)-7-[(1R,2S,3R)-2{4(R,S)-hydroxy-5,5-difluorooctyl}-3-methyl-5-oxocylopentyl]hept-5-enoate (31)

Hydrofluoric acid (1ml) was added to a solution of the compound (30) (0.201g) in acetonitrile (20ml). The resultant mixture was stirred at room temperature for 1 hour. The crude product obtained after the usual work-up was subjected to silicagel column chromatography to give the alcohol (31).
Yield: 0.138g (88%)

### 4-7) Preparation of methyl (Z)-7-[(1R,2S,3R)-2-(5,5-difluoro-4-oxooctyl)-3-methyl-5-oxocylopentyl]hept-5-enoate (32)

Celite (5g) was added to Collins reagent prepared from chromic anhydride (1.2g) and pyridine in methylene chloride (20ml), followed by addition of a solution of the alcohol (31) (0.138g) in methylene chloride (10ml). The resultant mixture was stirred at room temperature for 30 minutes, followed by the usual work-up. The obtained crude product was subjected to silicagel column chromatography to give the titled compound (32).
Yield: 81%
Compound (32) (L'=methyl, X₁=X₂=F, R₂-R₃=propyl, R'₁=methyl)
¹HNMR (CDCl₃) δ 0.97(t,3H,J=7.5Hz),1.13(d,3H,J=6Hz), 1.35-2.80(m,23H),3.67(s,3H),5.23-5.50(m,2H).
MS (DI-ZI) m/z 400(M⁺),369(M⁺-CH₃O)

### Example 5

### Preparation of 15-dehydroxy-17,17-difluoro-13,14-dihydro-16-oxo-PGE₁ methyl ester (32) [The IUPAC nomenclature: methyl 7-{(IR)-(2R,3S)-2-(5,5-difluoro-4-oxooctyl)-3-hydroxy-5-oxocylopentyl}hept-5-enoate]

### 5-1) Preparation of 7-[(1R)-(2R,3S,5S)-2-{5,5-difluoro-4(R,S)-hydroxyoctyl}-5-hydroxy-3-tetrahydropyranyloxycylopentyl]heptanoate (33)

Palladium on carbon (Pd-C) (100mg) was added to a solution of the diol (18) (0.465g) in ethyl acetate (30ml). The resultant mixture was stirred overnight under a hydrogen atmosphere. The reaction mixture was filtered and the filtrate was concentrated under reduced pressure to give the dihydro compound (33).
Yield: 0.450g (98%)

### 5-2) Preparation of methyl 7-{(1R)-(2R,3R)-2-(5,5-difluoro-4oxooctyl)-5-oxo-3-tetrahydropyranyloxycylopentyl} heptanoate (34)

Celite (10g) was added to Collins reagent prepared from chromic anhydride (3.67g) in methylene chloride (20ml), followed by addition of the dihydro compound (33) (0.450g) to be oxidized. The crude product obtained after the usual work-up was subjected to silicagel column chromatography to give the diketone (34).
Yield: 0.371g (83%)

### 5-3) Preparation of methyl 7-{(1R)-(2R,3R)-2-(5,5-difluoro-4-oxooctyl)-3-hydroxy-5-oxocylopentyl}heptanoate (35)

The diketone (34) (0.371g) was dissolved in a mixed solvent of acetic acid, THF and water (1:3:1, 35ml), and the resultant solution was stirred overnight. The crude product obtained after the usual work-up was chromatographed on a Rober column (ODS) to give the titled compound (35).
Compound (35) (X₁=X₂=F, R₂-R₃=propyl, R'₁=methyl)
¹HNMR (CDCl₃) δ 0.98(t,3H,J=7.5Hz),1.11-2.9(m,26H), 3.67(s,3H),4.1-4.25(m,1H).
MS (DI-ZI) m/z 404(M⁺),386(M⁺-H₂O),355(M⁺-H₂O-CH₃O)

### Example 6

### Preparation of 15-dehydroxy-17,17-difluoro-13,14-dihydro-16-oxo-PGD₂ methyl ester (43) [The IUPAC nomenclature: methyl (Z)-7-{(1R)-(2R,5S)-2-(5,5-difluoro-4-oxooctyl)-5-hydroxy-3-oxocylopentyl}hept-5-enoate]

### 6-1) Preparation of methyl (Z)-7-[(1R)-(2R,3R,5S)-2-{4(R,S)-t-butyldimethylsiloxy-5,5-difluorooctyl}-3,5-dihydroxycylopentyl]heptanoate (37)

The lactone (13) (1.06g) in toluene cooled to -78°C was reducted With DIBAL-H (1.5M in toluene, 7.56ml). The reaction mixture was worked up with the conventional procedure to give the lactol (36). Pottasium butoxide (1.0M in THF, 30.2ml) was added to a suspension of (4-carboxybutyl)triphenylphosphonium bromide (6.7g) in THF and the resultant mixture was stirred at room temperature for 30 minutes, and then cooled to -40°C. A solution of the lactol (36) in THF (15ml) was added thereto, and the mixture was stirred overnight at -20°C. The crude carboxylic acid obtained after the usual work up was esterified with diazomethane and the reaction mixture was subjected to silicagel column chromatography to give the diol (37).
Yield: 1.12g (85%)

### 6-2) Preparation of methyl (Z)-7-[(1R)-(2R,3R,5S)-2-{4(R,S)-t-butyldimethylsiloxy-5,5-difluorooctyl}-3-bezoyloxy-5-hydroxycylopentyl]hept-5-enoate (38)

A solution of the diol (37) (0.564g) and pyridine (0.85ml) in methylene chloride was cooled to -30°C. Benzoyl chloride (0.147g) was added thereto and the mixture was stirred for 1 hour. An additional amount (0.440g) of benzoyl chloride was added to the reaction mixture and the mixture was stirred at -20°C for 2 hours. The crude product obtained after the usual work-up was subjected to silicagel chromatography to give the compound (38).
Yield: 0.567g (77%)

### 6-3) Preparation of methyl (Z)-7-[(1R)-(2R,3R,5S)-2-{4(R,S)-t-butyldimethylsiloxy-5,5-difluorooctyl}-3-bezoyloxy-5-tetrahydropyranyloxycylopentyl]hept-5-enoate (39)

Dyhydropyran (0.6ml) was added to a solution of monobezoate compound (38) (0.567g) in methylene chloride and the resultant mixture was cooled to 0°C. A catalytic amount of p-toluenesulfonic acid was added thereto and the mixture was stirred for 30 minutes. The crude product obtained after the usual work-up was subjected to silicagel column chromatography to give the titled compound (39).
Yield: 0.689g

### 6-4) Preparation of methyl (Z)-7-[(1R)-(2R,3R,5S)-2-{4(R,S)-t-butyldimethylsiloxy-5,5-difluorooctyl}-3-hydroxy-5-tetrahydropyranyloxycylopentyl]hept-5-enoate (40)

Potassium carbonate (0.125g) was added to a solution of the compound (39) (0.689g) in methanol, and the resultant mixture was stirred at room temperature for 2 hours. An additional amount (1.75g) of potassium carbonate was added thereto, and the mixture was left on standing overnight. The crude product obtained after the usual work-up was subjected to silicagel column chromatography to give the monoalcohol (40).
Yield: 0.479g (87%, started from the compound (38))

### 6-5) Preparation of methyl (Z)-7-[(1R)-(2R,3R,5S)-2-{4(R,S)-hydroxy-5,5-difluorooctyl}-3-hydroxy-5-tetrahydro pyranyloxycylopentyl]hept-5-enoate (41)

Tetrabutylammonium fluoride (1.0M in THF, 3.95ml) was added to a solution of the monoalcohol (40) (0.479g) in THF and the mixture was stirred overnight at room temperature. The crude product obtained after the usual work-up was subjected to silicagel column chromatography to give the diol (41).
Yield: 72%

### 6-6) Preparation of methyl (Z)-7-{(1R)-(2R,5S)-2-(5,5-difluoro-4oxooctyl)-3-oxo-5-tetrahydropyranyloxycylopentyl}hept-5-enoate (42)

A solution of oxalyl chloride (0.24ml) in methylene chloride was cooled to -78°C, followed by addition of DMSO (0.44ml). After 15 minutes, a solution of the diol (41) (0.358g) in methylene chloride was added dropwise to the resultant mixture. After 30 minutes, the mixture was warmed to -50°C, followed by stirring for 1.5 hours. Then, the reaction mixture was allowed to warm to -35°C and triethylamine (0.88ml) was added thereto. The crude product obtained after the usual work-up was subjected to silicagel column chromatography to give the diketone (42).
Yield: 0.188g (53%)

### 6-7) Preparation of methyl (Z)-7-{(1R)-(2R,5S)-2-(5,5-difluoro-4oxooctyl)-5-hydroxy-3-oxocylopentyl}hept-5-enoate (43)

The diketone (42) (0.188g) was dissolved in a mixed solvent of acetic acid, THF and water (3:1:1, 25ml) and the resultant mixture was kept at 40°C for 3.5 hours. The crude product obtained after the usual work-up was subjected to silicagel column chromatography to give the titled compound (43).
Yield: 0.112g (72%)
Compound (43) (X₁=X₂=F, R₂-R₃=propyl, R'₁=methyl)
¹HNMR (CDCl₃) δ 0.98(t,3H,J=7.5Hz),1.4-2.8(m,22H), 3.69(s,3H),4.1-4.5(m,1H),5.4-5.6(m,2H).
MS (DI-ZI) m/z 402(M⁺),384(M⁺-H₂O),353(M⁺-H₂O-CH₃O) 333(M⁺-H₂O-CH₃O-HF)

### Example 7

### Preparation of 15-dehydroxy-17,17-difluoro-13,14-dihydro-16-oxo-PGA₂ methyl ester (45) [The IUPAC nomenclature: methyl (Z)-7-{(1R,2R)-2-(5,5-difluoro-4-oxooctyl)-5-oxocylopent-5-enyl}hept-5-enoate]

### 7-1) Preparation of methyl (Z)-7-[(1R,2R)-2-{5,5-difluoro-4-(R,S)-hydroxyoctyl}-5-oxocyclopent-3-enyl]hept-5-enoate (44)

The α,β-unsaturated ketone (29) (0.276g) was dissolved in a solution of aqueous hydrogen fluoride in acetonitrile (46% aqueous hydrogen fluoride:acetonitrile=95:5) (20ml), and the resultant mixture was stirred at room temperature for 2 hours. The crude product obtained after the usual work-up was subjected to silicagel column chromatography to give the alcohol (44).
Yield: 0.180g

### 7-2) Preparation of methyl (Z)-7-[(1R,2R)-2-{5,5-difluoro-4-oxooctyl}-5-oxocyclopent-3-enyl]hept-5-enoate (45)

Oxalyl chloride (2M in CH₂Cl₂) (0.47ml) was dissolved in methylene chloride (12ml), followed by addition of DMSO (0.12ml). The resultant mixture was cooled to -78°C and a solution of alcohol (44) (0.180g) in methylene chloride (10ml) was added. The mixture was stirred at -50°C for 1 hour. Then, triethylamine (0.23ml) was added thereto, and the resultant mixture was stirred at -30°C for 30 minutes.

The crude product obtained after the usual work-up was subjected to silicagel column chromatography to give the titled compound (45).
Yield: 0.126g (71%)
Compound (45) (X₁=X₂=F, R₂-R₃=propyl, R'₁=methyl)
¹HNMR (CDCl₃) δ 1.00(t,3H,J=7.5Hz),1.40-2.80(m,20H), 3.70(s,3H),5.28-5.55(m,2H),6.17(dd,1H,J=7.5,J=2.5),7.63(dd, 1H,J=7.5,J=2.5).
MS (DI-ZI) m/z 384(M⁺),353(M⁺-CH₃O)

### Example 8

### Preparation of 15-dehydroxy-17,17-difluoro-13,14-dihydro-16-oxo-PGF₂α methyl ester (49) [The IUPAC nomenclature: methyl (Z)-7-{(1R)-(2R,3R,5S)-2-(5,5-difluoro-4-oxooctyl)-3,5-dihydroxycylopentyl}hept-5-enoate]

### 8-1) Preparation of methyl (Z)-7-[(1R)-(2R,3R,5S)-2-{4(R,S)-t-butyldimethylsiloxy-5,5-difluorooctyl}-3,5-ditetrahydropyranyloxy)cylopentyl]hept-5-enoate (46)

A solution of methyl (Z)-7-[(1R)-(2R,3R,5S)-2-{4(R,S)-t-butyidimethylsiloxy-5,5-difluorooctyl}-3,5-dihydroxy-cylopentyl]-hept-5-enoate (27) (0.647g) in dichloromethane (10ml) was cooled to -5°C, followed by addition of dihydropyran (0.91ml) and a catalytic amount of p-toluenesulfonic acid. The reaction mixture was gradually warmed to room temperature and kept for 16 hours at the same temperature. The crude product obtained after the usual work-up was subjected to silicagel column chromatography to give the titled compound (46).
Yield: 0.893g (100%)

### 8-2) Preparation of methyl (Z)-7-[(1R)-(2R,3R,5S)-2-{5,5-difluoro-4(R,S)-hydroxyooctyl}-3,5-di(tetrahydropyranyloxy)cyclopentyl]hept-5-enoate (47)

Tetrabutylammonium fluoride (1M in THF, 3.72ml) was added to a solution of the compound (46) (0.89g) in THF (12ml) and the resultant mixture was stirred for 1 hour. The crude product obtained after the usual work-up was subjected to silicagel column chromatography to give the titled compound.
Yield: 0.676g (95%)

### 8-3) Preparation of methyl (Z)-7-[(1R)-(2R,3R,5S)-2-{5,5-difluoro-4-oxooctyl}-3,5-di(tetrahydropyranyloxy)cylopentyl]hept-5-enoate (48)

The compound (47) (0.43ml) was oxidized by Swarn oxidation using 2M oxalyl chloride (0.76ml), DMSO (0.22ml) and triethylamine (0.43ml) in dichloromethane (9ml). The crude product obtained after the usual work-up was subjected to silicagel column chromatography to give the titled compound (48).
Yield: 0.558g (82%)

### 8-4) Preparation of methyl (Z)-7-[(1R)-(2R,3R,5S)-2-{5,5-difluoro-4-oxooctyl}-3,5-di(tetrahydropyranyloxy)cylopentyl]hept-5-enoate (49)

The compound (48) (0.558g) was dissolved in a mixed solvent of acetic acid, water and THF (4:2:1, 49ml), and the resultant solution was kept at 45 to 50°C for 2.5 hours. The crude product obtained after the usual work-up was chromatographed on a silicagel column to give the titled compound (49).
Yield: 0.367g (94%)
Compound (49) (X₁=X₂=F, R₂-R₃=propyl, R'₁=methyl)
¹HNMR (CDCl₃) δ 0.95(t,3H),1.1-3.0(m,24H),3.66(s,3H), 3.95(s,1H),4.14(s,1H),5.28-5.52(m,2H).
MS (DI-ZI) m/z 404(M⁺),386(H⁺-H₂O),368(H⁺-2H₂O)

### Example 9

### Preparation of 15-dehydroxy-17,17-difluoro-13,14-dihydro-20-methyl-16-oxo-PGE₂ methyl ester (20) [The IUPAC nomenclature: methyl (Z)-7-{(1R)-(2R,3R)-2-(5,5-difluoro-4-oxononyl)-5-oxo-3-hydroxycylopentyl}hept-5-enoate]

The titled compound (20) was prepared from the compound (8) and dimethyl (3,3-difluoro-2-oxoheptyl)phosphonate according to the procedure described for the preparation of 15-dehydroxy-17,17-difluoro-13,14-dihydro-20-methyl-16-oxo-PGE₂ methyl ester.
Compound (20) (X₁=X₂=F, R₂-R₃=butyl, R'₁=methyl)
¹HNMR (CDCl₃) δ 0.94(t,3H),1.1-2.9(m,27H), 3.68(s,3H),4.2(br.s,1/2H),4.4(q,1/2H),5.4(m,2H).
MS (DI-ZI) m/z 384(M⁺-H₂O),353(M⁺-H₂O-CH₃O).

### Example 10

### Preparation of 15-dehydroxy-17,17-difluoro-13,14-dihydro-16-oxo-PGE₁ isopropyl ester (35) [The IUPAC nomenclature: isopropyl 7-{(1R)-(2R,3R)-2-(5,5-difluoro-4-oxooctyl)-3-hydroxy-5-oxocylopentyl}hept-5-enoate]

A solution of 15-dehydroxy-17,17-difluoro-13,14-dihydro-16-oxo-PGE₂ isopropyl ester (20) (0.303g) obtained in Example 3 in ethyl acetate (20ml) was subjected to hydrogenation with a catalytic amount of 5% Pd-C and hydrogen gas. The reaction mixture was filtered and the filtrate was concentrated to give the crude product, which was chromatographed on a Rober column to give the titled compound (35).
Yield: 0.223g (73%)
Compound (35) (X₁=X₂=F, R₂-R₃=propyl, R'₁=isopropyl)
¹HNMR (CDCl₃) δ 0.98(t,3H,J=7.5Hz),1.21(d,6H,J=5.5Hz), 1.24-2.82(m,27H),4.1-4.5(m,1H),4.99(Hept,1H,J=7.5Hz).

### Example 11

### Preparation of 15-dehydroxy-17,17-difluoro-13,14-dihydro-16-oxo-PGE₂ benzyl ester (20) [The IUPAC nomenclature: benzyl (Z)-7-{(1R)-(2R,3R)-2-(5,5-difluoro-4-oxooctyl)-3-hydroxy-5-oxocylopentyl}hept-5-enoate]

The titled compound (20) was prepared as described in Example 3 except that the crude carboxylic acid (16) in acetonitrile was converted to benzyl ester using benzyl bromide and DBU.
Compound (20) (X₁=X₂=F, R₂-R₃=propyl, R'₁=benzyl)
¹HNMR (CDCl₃) δ 0.96(d,t,3H,J=7.5Hz,J=7.5Hz), 1.1-2.8(m,23H),4.18(m,0.7H),4.36(m,0.3H),5.11(s,2H), 5.38(m,2H),7.35(s,5H).

### Example 12

### Preparation of 15-dehydroxy-17,17-difluoro-13,14-dihydro-16-oxo-PGE₁ [The IUPAC nomenclature: 7-{(1R)-(2R,3R)-2-(5,5-difluoro-4-oxooctyl)-3-hydroxy-5-oxocylopentyl}heptanoic acid]

The benzyl ester (20) (0.580g) obtained in Example 11 was subjected to catalytic hydrogenation in ethanol (20ml) using 5% Pd-C (a catalytic amount) and hydrogen gas. The obtained crude product was purified with HPLC (OD column) to give the titled compound.
Yield: 0.426g (90%)
¹HNMR (CDCl₃) δ 0.98(t,3H,7.5Hz),1.1-2.82(m,28H), 4.07-4.45(m,1H).

The compound having Formula (I) wherein Y is -CO-CH₂- or Y is -C=C- can be prepared as follows.

### Example 13

### Preparation of 15-dehydroxy-13,14-dihydro-6,16-dioxo-PGF₁α isopropyl ester (52)

To a solution of 15-dehydroxy-13,14-dihydro-16,16-ethylenedioxy-11-tetrahydropyranyloxy-PGF₂α (50) in a mixed solvent of tetrahydrofuran and methylene chloride was added N-bromosuccinimide equimolar to the compound (50). The resultant mixture was stirred for 5 minutes. The crude product obtained after the usual work-up was chromatographed on a silicagel column to give the compound (51) (X₁=X₂=H, R₂-R₃=propyl, P₄=tetrahydropyranyl, P₇=ethylene, R'₁=isopropyl). DBU was added to a solution of the compound (50) in toluene, and the resultant mixture was stirred overnight at 40°C. After cooling with ice, the reaction mixture was acidified with 1N hydrochloric acid. After stirring for 10 minutes, the solution was extracted with ethyl acetate. The crude product obtained after the usual work-up was chromatographed on a silicagel column to give the titled compound (52) (the symbols have the same meanings as above).

### Example 14

### Preparation of 15-dehydroxy-5,6-dehydro-13,14-dihydro-16-oxo-PGE₂ methyl ester

t-Butyllithium was added dropwise over 30 minutes to a solution of 4,4-ethylenedioxyoctane iodide in ether at -78°C, and the resultant mixture was stirred for 3 hours. A solution of copper(I) iodide and tributylphosphine in ether, previously cooled at -78°C, was added in one portion. The reaction mixture was stirred for 20 minutes to produce a complex (a). Further, a solution of 4R-t-butyldimethylsilyloxy-2-cyclopenten-1-one (53) in tetrahydrofuran was added dropwise thereto over 95 minutes, and stirring was continued for 15 minutes. The resultant mixture was transferred in a cooling bath at -30°C. A solution of 8-methoxycarbonyl-2-hexynyl-1-iodide (b) in HMPA was added thereto, and the resultant mixture was stirred at same temperature for 4.5 hours, followed by stirring for additional 12 hours at room temperature. The reaction mixture was poured into saturated aqueous ammonium chloride solution and the organic phase was separated. The crude product obtained after the usual work-up was chromatographed to give the compound (54) (X₁=X₂=H, R₂-R₃=propyl, P₆=t-butyldimethylsilyl, P⁷=ethylene, R'₁=isopropyl), which was deblocked in the usual work-up to give the titled compound.

## Claims

1. A compound of the formula: 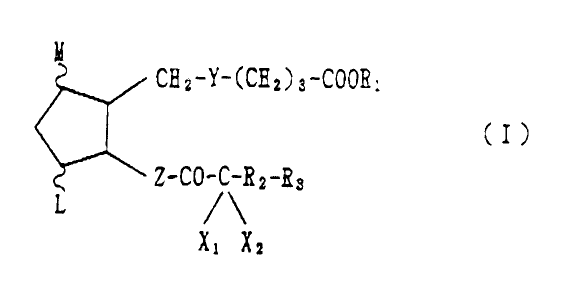 wherein
L and M are hydrogen atom, hydroxy, lower alkyl, hydroxy(lower)alkyl or oxo, provided that at least one of L and M is not hydrogen atom and that the five-membered ring may have one or two double bonds,
X₁ and X₂ are hydrogen atom, halogen atom or lower alkyl,
Y is -CH₂-CH₂-, -CH=CH-, -C≡C- or -CO-CH₂-,
Z is -CH₂-CH₂-CH₂-, -CH=CH-CH₂ or -CH₂-CH=CH-,
R₁ is hydrogen atom, lower alkyl, lower cycloalkyl, monocyclic aryl, monocyclic aryl(lower)alkyl or monocyclic aroyl(lower)alkyl,
R₂ is single bond or lower alkylene,
R₃ is lower alkyl which is unsubstituted or substituted with halogen, lower cycloalkyl which is unsubstituted or substituted with lower alkyl, monocyclic aryl which is unsubstituted or substituted with halogen or halo(lower)alkyl, or monocyclic aryloxy which is unsubstituted or substituted with halogen or halo(lower)alkyl,
or a pharmaceutically acceptable salt when R₁ is hydrogen atom.

2. A compound according to claim 1, in which X₁ is fluorine atom.

3. A compound according to claim 2, in which X₂ is fluorine atom.

4. A compound according to claim 1, in which Y is -CH=CH.

5. A compound according to claim 1, in which Z is -CH₂-CH₂-CH₂-.

6. A compound according to claim 1, in which R₂ is single bond and R₃ is lower alkyl.

7. A compound according to claim 1, in which L and M are hydrogen atom, hydroxy or oxo and the five-membered ring may have one double bond, X₁ and X₂ are hydrogen atom, fluorine atom or methyl, Y is -CH₂-CH₂-, -CH=CH-, -C≡C- or -CO-CH₂-, Z is -CH₂-CH₂-CH₂- or -CH=CH-CH₂-, R₁ is hydrogen atom, methyl, ethyl, isopropyl, benzyl or phenacyl, and R₂-R₃ is butyl or hexyl.

## Patentansprüche

1. Verbindung der Formel 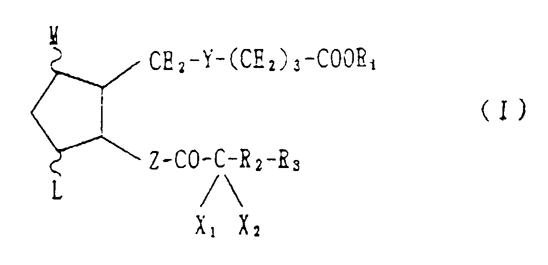 worin
L und M ein Wasserstoffatom, Hydroxy, Niedrigalkyl, Hydroxy(niedrig)alkyl oder Oxo bedeuten, mit der Maßgabe, daß mindestens einer der Substituenten L und M nicht ein Wasserstoffatom bedeutet und daß der fünfgliedrige Ring eine oder zwei Doppelbindungen aufweist,
X₁ und X₂ ein Wasserstoffatom, ein Halogenatom oder Niedrigalkyl bedeuten,
Y -CH₂-CH₂-, -CH=CH-, -C≡C- oder -CO-CH₂- bedeutet,
Z -CH₂-CH₂-CH₂-, -CH=CH-CH₂- oder -CH₂-CH=CH- bedeutet,
R₁ ein Wasserstoffatom, Niedrigalkyl, Niedrigcycloalkyl, monocyclisches Aryl, monocyclisches Aryl(niedrig)alkyl oder monocyclisches Aroyl(niedrig)alkyl bedeutet,
R₂ eine Einfachbindung oder Niedrigalkylen bedeutet,
R₃ Niedrigalkyl, welches unsubstituiert ist oder substituiert ist mit Halogen, Niedrigcycloalkyl, welches unsubstituiert ist oder substituiert ist mit Niedrigalkyl, monocyclisches Aryl, welches unsubstituiert ist oder substituiert ist mit Halogen oder Halo(niedrig)alkyl, oder monocyclisches Aryloxy, welches unsubstituiert ist oder substuiert mit Halogen oder Halo(niedrig)alkyl, bedeutet,
oder ein pharmazeutisch annehmbares Salz davon, wenn R₁ ein Wasserstoffatom bedeutet.

2. Verbindung nach Anspruch 1, worin X₁ ein Fluoratom bedeutet.

3. Verbindung nach Anspruch 2, worin X₂ ein Fluoratom bedeutet.

4. Verbindung nach Anspruch 1, worin Y -CH=CH-bedeutet.

5. Verbindung nach Anspruch 1, worin Z -CH₂-CH₂-CH₂-bedeutet.

6. Verbindung nach Anspruch 1, worin R₂ eine Einfachbindung und R₃ Niedrigalkyl bedeuten.

7. Verbindung nach Anspruch 1,worin L und M Wasserstoffatome, Hydroxy oder Oxo bedeuten und der fünfgliedrige Ring eine Doppelbindung aufweisen kann, X₁ und X₂ ein Wasserstoffatom, ein Fluoratom oder Methyl bedeuten, Y -CH₂-CH₂-, -CH=CH-, -C≡C- oder -CO-CH₂- bedeutet, Z -CH₂-CH₂-CH₂- oder -CH=CH-CH₂- bedeutet, R₁ ein Wasserstoffatom, Methyl, Ethyl, Isopropyl, Benzyl oder Phenacyl bedeutet und R₂-R₃ Butyl oder Hexyl bedeuten.

## Revendications

1. Composé de la formule : 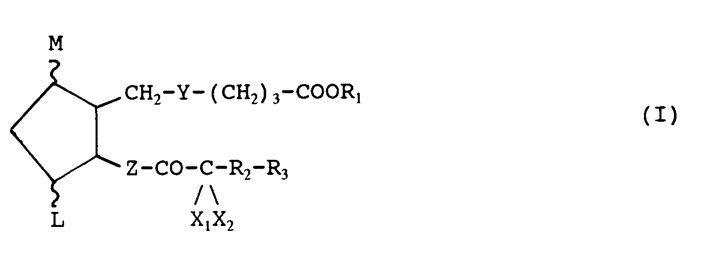 dans laquelle
L et M représentent des atomes d'hydrogène, des radicaux hydroxyle, alkyle inférieur, hydroxyalkyle(inférieur) ou oxo,
avec la condition qu'au moins l'un des symboles L et M ne représente pas d'atome d'hydrogène et que le noyau pentagonal puisse comporter une ou deux doubles liaisons,
X₁ et X₂ représentent des atomes d'hydrogène, des atomes d'halogène ou des radicaux alkyle inférieurs,
Y est -CH₂-CH₂-, -CH=CH-, -C≡C- ou -CO-CH₂-,
Z est -CH₂-CH₂-CH₂-, -CH=CH-CH₂ ou -CH₂-CH=CH-,
R₁ représente un atome d'hydrogène, un radical alkyle inférieur, cycloalkyle inférieur, aryle monocyclique, monocycloarylalkyle(inférieur) ou monocycloaroylalkyle(inférieur),
R₂ représente une simple liaison ou un radical alkylène,
R₃ représente un radical alkyle inférieur qui n'est pas substitué ou qui est substitué par des atomes d'halogène, un radical cycloalkyle inférieur qui n'est pas substitué ou qui est substitué par des radicaux alkyle inférieurs, un radical aryle monocyclique qui n'est pas substitué ou qui est substitué par des halogènes ou des radicaux haloalkyle inférieurs ou aryloxymonocyclique qui n'est pas substitué ou qui est substitué par des halogènes ou des radicaux haloalkyle inférieurs,
ou un sel pharmaceutiquement acceptable d'un tel composé lorsque R₁ représente un atome d'hydrogène.

2. Composé suivant la revendication 1, caractérisé en ce que X₁ représente un atome de fluor.

3. Composé suivant la revendication 2, caractérisé en ce que X₂ représente un atome de fluor.

4. Composé suivant la revendication 1, caractérisé en ce que Y représente un groupe -CH=CH.

5. Composé suivant la revendication 1, caractérisé en ce que Z représente un groupe -CH₂-CH₂-CH₂-.

6. Composé suivant la revendication 1, caractérisé en ce que R₂ représente une simple liaison et R₃ représente un radical alkyle inférieur.

7. Composé suivant la revendication 1, caractérisé en ce que L et M représentent des atomes d'hydrogène, des radicaux hydroxyle, ou oxo et le noyau pentagonal peut comporter une double liaison, X₁ et X₂ représentent des atomes d'hydrogène, des atomes de fluor ou des radicaux méthyle, Y est -CH₂-CH₂-, -CH=CH-, -C≡C- ou -CO-CH₂-, Z est -CH₂-CH₂-CH₂- ou -CH=CH-CH₂-, R₁ représente un atome d'hydrogène, un radical méthyle, éthyle, isopropyle, benzyle ou phénacyle, et R₂-R₃ représente le radical butyle ou hexyle.
